(19) Europäisches Patentamt
European Patent Office
Office européen des brevets

(11) Publication number : 0 275 065 B1

(12) # EUROPEAN PATENT SPECIFICATION

(45) Date of publication of patent specification : 19.06.91 Bulletin 91/25

(51) Int. Cl.⁵ : C12N 1/20, C12N 9/10, // C12P19/40 , (C12N1/20, C12R1:13)

(21) Application number : 88100254.7

(22) Date of filing : 29.03.83

(54) **New phosphorylase producing microorganism.**

(30) Priority : 01.04.82 JP 55078/82
04.11.82 JP 194394/82

(43) Date of publication of application : 20.07.88 Bulletin 88/29

(61) Publication number of the earlier application in accordance with Art. 76 EPC : 0090417

(45) Publication of the grant of the patent : 19.06.91 Bulletin 91/25

(84) Designated Contracting States : CH DE FR GB LI

(56) References cited :
EP-A- 0 093 401
GB-A- 1 063 952

(73) Proprietor : Yamasa Shoyu Kabushiki Kaisha
10-1, Araoi-Cho 2-Chome
Choshi-Shi Chiba-Ken 288 (JP)

(72) Inventor : Fujishima, Tetsuro
8539-8, Kobatake Shin Machi
Choshi-Shi Chiba-Ken (JP)
Inventor : Sakata, Shinji
2-2, Sakae-Cho 2-chome
Choshi-Shi Chiba-Ken (JP)

(74) Representative : Dr. Elisabeth Jung Dr. Jürgen Schirdewahn Dipl.-Ing. Claus Gernhardt
P.O. Box 40 14 68 Clemensstrasse 30
W-8000 München 40 (DE)

EP 0 275 065 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Phosphorylase producing microorganisms are useful for carrying out certain enzymatic reactions.

A specific reaction of this kind is used in the process for producing 3'-deoxyguanosine from a guanine derivative represented by the formula (I) :

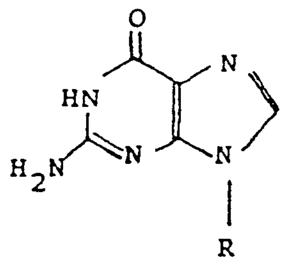

[I]

wherein R designates a hydrogen atom or a ribose-1-yl group, a 2-deoxyribose-1-yl group or a monophosphate, a diphosphate or a triphosphate thereof, by reacting it with a 3-deoxyribose donor in the presence of a nucleoside phosphorylase source.

This process is described and claimed in the parent application No. 83 103 128.1.

A new phosphorylase producing strain of the genus Brevibacterium (hereinafter referred to as Br.) has been isolated from the sand in the baseball ground of Koshien, Nishinomiya-Shi, Hyogo-Ken, Japan.

This strain has the following characteristic bacteriological features :

A. Morphology :

(1) Form and size of cells : short rod-shaped,

0.8 − 1.0 × 1.0 − 1.2 μm ;
(2) Formation of spores : none
(3) Gram staining : positive

B. Growth on various culture media :

(1) Bouillon-agar plate culture (28°C, 48 hours)
① Form of colony : circular
② Raising of colony surface : flat, smooth
③ Size : 2-4 mm
④ Color tone : yellow to peach-yellow
(2) Bouillon-agar slant culture (28°C, 48 hours)
① Growth : good
② Form of growth : echinulate
(3) Bouillon liquid culture (28°C, 48 hours)
Growth : formation of ring on the surface, sediment slightly formed.
(4) Bouillon-gelatin stab culture (20°C, 6 days) : liquefied in stratiform
(5) Litmus-milk culture medium (28°C, 4 days) : slightly coagulated, peptonization also observed.

C. Physiological properties :

(1) Reduction of a nitrate (28°C, 5 days) : no reductivity
(2) Formation of hydrogen sulfide (28°C, 5 days) : not formed
(3) Hydrolysis of starch : hydrolyzed
(4) Catalase : positive
(5) Indole formation : not formed
(6) Ammonia formation from peptone and arginine : negative
(7) Methyl red test : negative
(8) V-P test : positive
(9) Attitude to oxygen : aerobic

(10) O-F test (by the Hugh Leifson method) : F type (Fermentation)
(11) Acid formation from sugars
positive : glucose, mannose, fructose, maltose, saccharose and trehalose ;
negative : arabinose, xylose, galactose, lactose, sorbitol, inositol and glycerine
(12) Growth pH range : pH 6.0-9.0
(13) Optimum growth temperature : 25-37°C

The above bacteriological properties were examined with reference to the taxonomical standards in Bergey's Manual of Determinative Bacteriology, 7th edition (1957).

The strain, which is a short-rod bacterium almost approximate to a coccus, Gram-positive, forms no filament and forms acids from carbohydrates, was identified to be a strain belonging to the genus Brevibacterium and designated Brevibacterium acetylicum AT-6-7.

Although the above microorganism strain was identified according to Bergey's Manual of Bacteriology, 7th edition, it is possible that it may belong to another species or genus when this strain is to be identified to belong to another species or genus according to different taxonomical standards due to some changes in taxonomical standards in the future. However, the microorganism as designated above can at least produce nucleoside phosphorylase in conformity with the object of the present invention and has the aforesaid bacteriological properties or bacteriological properties equivalent thereto, and can be unequivocally specified.

This microorganism strain was deposited at the Fermentation Research Institute, Agency of Industrial Science & Technology on January 13, 1982, under the following deposition number (FERM P. No.). Further, this strain was sent directly from the Fermentation Research Institute to the American Type Culture Collection (ATCC), 12301 Parklawn Drive, Rockville, Maryland, U.S.A. for deposition, and deposited on March 2, 1983, under the following deposition number (ATCC No.) :

Brevibacterium acetylicum AT-6-7, FERM P-6305, ATCC 39311. The present invention also refers to new phosphorylase producing mutant strains derived from the above defined microorganism strain Brevibacterium acetylicum AT-6-7. Such mutant strains may be obtained through induced mutation according to the mutagenic methods in general by a physical treatment such as irradiation of UV-ray, X-ray or V-ray or by a chemical treatment with nitrosoguanidine or other mutagens or by natural mutation attributable to natural causes. Such mutant strains falls into the scope of the present invention, so long as they do not lose the ability to produce nucleoside phosphorylase.

In cultivation of this microorganism in order to produce a nucleoside phosphorylase, the culture medium and the method of culture employed are not particularly limited, so far as growth of this microorganism is concerned.

As a culture medium, there may be employed one containing appropriate amounts of a carbon source and a nitrogen source assimilable by these microorganisms, optionally added with an inorganic salt, minute amounts of a growth promoter, defoaming agents, etc. More specifically, as carbon sources, there may be employed one or more of those selected suitably in view of assimilability by the microorganism employed from carbon sources in general, including sugars such as glucose, fructose, maltose, galactose, ribose, saccharose, starch, starch hydrolysate, molasses, waste molasses, etc. or derivatives thereof such as fatty acid esters thereof ; natural carbohydrates such as wheat, wheat bran, rice, etc. ; alcohols such as glycerol, mannitol, methanol, ethanol, etc. ; fatty acids such as gluconic acid, pyruvic acid, acetic acid, citric acid, etc. ; hydrocarbons such as normal paraffins kerosene, etc. ; amino acids such as glycine, glutamic acid, glutamine, alanine, asparagine, etc. ; and so on. As nitrogen sources, there may be employed one or more of those selected suitably in view of assimilability by the microorganism employed from nitrogen sources in general, including organic nitrogenous materials such as meat extract (bouillon), peptone, yeast extract, dry yeast, soybean hydrolysate, soybean powder, milk casein, casamino acid, various amino acids, corn steep liquor, cotton seed meal or its hydrolysate, fish meal or its hydrolysate, hydrolysates of other animals, vegetables, microorganisms, etc. ; inorganic nitrogen compounds such as ammonia, ammonium salts such as ammonium nitrate, ammonium sulfate, ammonium chloride, ammonium phosphate, ammonium carbonate, ammonium acetate and the like, nitric acid salts such as sodium nitrate, urea, and so on. Further, as inorganic salts, there may suitably be added one or more minute amounts, of phosphates, hydrochlorides, sulfates, carbonates, nitrates, acetates and others of magnesium, manganese, iron, zinc, copper, sodium, calcium, potassium, etc. If necessary, there may also be added a defoaming agent such as a vegetable oil or a surfactant, a minute amount of a growth promoter such as vitamins $B_1$, $B_2$, nicotinic acid, pantothenic acid, biotin, p-aminobenzoic acid, etc..

Cultivation may be performed in a liquid medium containing the above culture medium components by selecting a culture method suitable for the micoorganism from conventional culture methods such as shaking culture, aerating stirring culture, stationary culture, continuous culture and others.

The cultural conditions may be suitably chosen depending on the culture medium employed, but generally

by adjusting before start-up of cultivation at pH of about 6 to 8 and carrying out cultivation under the temperature condition of about 25 to 35°C. The culture duration may be a period sufficient for growth of the microorganism, being generally 1 to 3 days.

After culturing the microorganism as described above, the culture, the intact microbial cells collected from the culture according to a conventional method such as centrifugation, sedimentation separation, agglomeration separation, or a modification of microbial cells obtained by applying a suitable treatment on the living or intact cells may be used as the nucleoside phosphorylase source useful for the enzymatic reactions. The "culture" herein refers to a product under the state where the culture medium and the cultured microbial cells after cultivation are still unseparated from each other. The "modification of microbial cells" refers to dried microbial cells, microbial cells whose cell wall and/or membrane having been modified, crushed microbial cells, immobilized microbial cells, extracts of microbial cells, protein fractions having nucleoside phosphorylase activity of extract of microbial cells or purified product thereof, immobilized product of the protein fractions or purified product thereof, and the like. Modifications of microbial cells can be obtained by applying on intact microbial cells singly or in combination physical treatment means such as freezing-thawing, lyophilization, air drying, acetone drying, heating under acidic or alkaline conditions, grinding, ultrasonic treatment, osmotic treatment, etc. or chemical or biochemical treatments such as enzymatic treatments with lysozyme, cell wall lysing enzymes, etc., contact treatments with solvents such as toluene, xylene, butyl alcohol or surfactants, or by applying on the extract of microbial cells singly or in combination enzyme separation and purification means such as salting-out, isoelectric point precipitation, precipitation with organic solvents, various chromatographies, dialysis and others, or further by applying on intact microbial cells, extracts of microbial cells or purified products thereof an enzyme or cell immobilization means such as inclusion method, crosslinking method, adsorption method onto a carrier, etc..

## Examples of the preferred embodiments

The present invention is to be described in further detail below by referring to Examples, each of which is illustrative of an embodiment of the present invention and not limitative of the scope of the present invention. In Examples, analysis of 3'-deoxyguanosine was conducted by high performance liquid chromatography. When analyzed by means of the device and under the conditions shown below, 3'-deoxyguanosine is eluted at a retention time around 12.90 minutes and its quantity can be calculated from the calibration curve.

Device : Shimadzu High Performance Liquid Chromatograph LC-3A model (produced by Shimadzu Corporation)

Column : Sorbax ODS, 4.6 mm × 250 mm (Shimadzu Du Pont Co.)

Eluant : 20 mM Tris-hydrochloric acid buffer containing 5% acetonitrile (pH 7.5)

Flow rate : 1 ml/minute

Column operation temperature : room temperature

## Example A1

Two liters of a 2% bouillon culture medium were sterilized at 120°C for 15 minutes and cooled. Then, 100 ml of a previously precultured culture broth of Brevibacterium acetylicum AT-6-7 (FERM P-6305) was added to the culture broth and cultivation was carried out at 28°C for 22 hours.

After completition of the cultivation, the cells were collected by centrifugation and added into 200 ml of a sterilized water to be suspended therein. Into 200 ml of a substrate solution (pH 7.0) containing 25 mM guanosine-5'-monophosphate (GMP)(disodium salt), 25 mM 3'-deoxyadenosine (3'-dAdo) and 30 mM monosodium dihydrogen phosphate was added the above cell suspension and the reaction was carried out at 60°C for 36 hours.

The reaction mixture after removal of the cells by centrifugation was analyzed by high performance liquid chromatography to show that the yield of 3'-deoxyguanosine was 36.58%. The yield of 3'-deoxyguanosine is defined as the molar ratio (%) of 3'-deoxyguanosine formed to 3'-deoxyadenosine added.

## Example A2

After cultivation of Brevibacterium acetylicum AT-6-7 as described in example A1, but using only each 100 ml of a bouillon medium, cells were collected and each sample was diluted with 10 ml of sterilized water to prepare cell suspensions.

To each suspension was added 10 ml of solutions containing 20 mM 3'-dAdo, 25 mM monopotassium dihydrogen phosphate and 20 mM GMP. The enzymatic reaction was carried out for 24 hours at different temperatures within the range of from 40 to 80°C. After the reaction, the supernatant obtained by centrifugation was analyzed to give the 3'-deoxyguanosine yield as shown in Table I.

Table I

| Reaction temperature | 3'-Deoxyguanosine yield (%) |
|---|---|
| 40°C | 5.81 |
| 50°C | 12.32 |
| 60°C | 20.86 |
| 70°C | 4.32 |
| 80°C | 0 |

## Claims

1. A new phosphorylase producing microorganism of the genus Brevibacterium, designated as Brevibacterium acetylicum AT-6-7 (FERM P-6305, ATCC 39311) being a short-rod bacterium, Gram-positive, forming no filament and forming acids from carbohydrates.
2. New phosphorylase producing mutant strains derived from Brevibacterium acetylicum AT-6-7.
3. New phosphorylase producing mutant strains according to claim 2 derived through induced mutation by mutagenic physical and/or chemical methods.
4. New phosphorylase producing mutant strains according to claim 2 derived through natural mutation.

## Ansprüche

1. Ein neuer Phosphorylase produzierender Mikroorganismus der Gattung Brevibacterium, genannt Brevibacterium acetylicum AT-6-7 (FERM P-6305, ATCC 39311), welcher ein Kurzstäbchen-Bakterium und Gram-positiv ist, keine Fäden bildet und aus Kohlehydraten Säuren bildet.
2. Neue Phosphorylase produzierende Mutantenstämme, welche von Brevibacterium acetylicum AT-6-7 abgeleitet sind.
3. Neue Phosphorylase produzierende Mutantenstämme nach Anspruch 2, welche durch induzierte Mutation mittels mutagener physikalischer und/oder chemischer Methoden abgeleitet worden sind.
4. Neue Phosphorylase produzierende Mutantenstämme nach Anspruch 2, welche durch natürliche Mutation abgeleitet worden sind.

## Revendications

1. Nouveau micro-organisme producteur de phosphorylase, du genre Brevibacterium, nommé Brevibacterium acetylicum AT-6-7 (FERM P-6305, ATCC 39311), bactérie qui a la forme d'un bâtonnet court, qui est Gram positive, qui ne forme pas de filaments et qui engendre des acides à partir de glucides.

2. Nouvelles souches mutantes productrices de phosphorylase, souches qui dérivent de Brevibacterium acetylicum AT-6-7.

3. Nouvelles souches mutantes productrices de phosphorylase selon la revendication 2, qui dérivent par mutation induite par des méthodes physiques et/ou chimiques mutagènes.

4. Nouvelles souches mutantes productrices de phosphorylase selon la revendication 2 qui dérivent par mutation naturelle.